# DEMANDE DE BREVET EUROPEEN

(11) **EP 0 550 313 A1**
(43) Date de publication de la demande: **07.07.1993**
(21) Numéro de dépôt: 92403477.0
(22) Date de dépôt: 18.12.1992
(51) Int. Cl.: C07D 257/04, C07D 405/10

(54) **Nouveaux dérivés de 2-(tétrazol-5-yl)-(1,1'-biphényle), leur préparation et leur utilisation comme intermédiaires de synthèse**

(30) Priorité: 30.12.1991 FR 9116290; 16.03.1992 FR 9203113
(71) Demandeur: SYNTHELABO, F-92350 Le Plessis Robinson (FR)
(72) Inventeur: Daumas, Marc, F-75013 Paris (FR); Hoornaert, Christian, F-75013 Paris (FR); Chekroun, Isaac, F-93800 Epinay (FR); Bedoya-Zurita, Manuel, F-75003 Paris (FR); Ruiz-Montes, José, F-78200 Mantes la Jolie (FR); Greciet, Hélène, F-27100 Val de Reuil (FR); Rossey, Guy, F-78960 Voisins-le-Bretonneux (FR)
(74) Mandataire: Thouret-Lemaitre, Elisabeth

(57) **Abrégé**

Composés répondant à la formule (I) 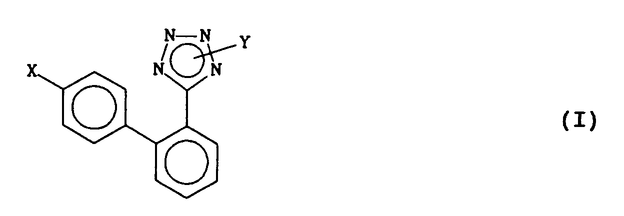
dans laquelle
X représente soit un groupe dibromométhyle, soit un groupe formyle, soit un groupe (C₁₋₄)alkyle, soit un groupe CH(OR₅)₂, soit un groupe CH(OH)OR₅, R₅, étant un atome d'hydrogène ou un groupe (C₁₋₃)alkyle qui peut éventuellement former dans le cas de CH(OR₅)₂ un cycle 1,3-dioxolane ou 1,3-dioxane et
Y représente soit un atome d'hydrogène, soit un groupe 1,1-diméthyléthyle, soit un groupe triphénylméthyle, soit un groupe triméthylstannyle, soit un groupe tributylstannyle, soit un groupe (1,1-diméthyléthyl)diméthylsilyle, soit un groupe (1,1-diméthyléthyl)diphénylsilyle, soit un groupe 2-cyanoéthyle, soit un groupe CH₂OR₆ où R₆ est un groupe méthyle, phénylméthyle, 1,1-diméthyléthyle, 2,2,2-trichloroéthyle, benzyloxycarbonyle ou 2,2,2-trichloroéthyloxycarbonyle,
Y étant en position 1 ou 2 du cycle tétrazole.

Utilisation comme intermédiaires dans la synthèse de composés à activité thérapeutique.

## Description

La présente invention a pour objet des dérivés de 2-(tétrazol-5-yl)-[1,1'-biphényle] et leur préparation.

Les composés de l'invention répondent à la formule (I) 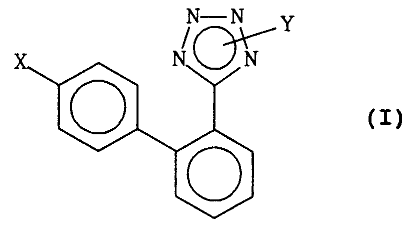
dans laquelle
X représente soit un groupe dibromométhyle, soit un groupe formyle, soit un groupe (C₁₋₄)alkyle, soit un groupe CH(OR₅)₂, soit un groupe CH(OH)OR₅, R₅ étant un atome d'hydrogène ou un groupe (C₁₋₃)alkyle qui peut éventuellement former dans le cas de CH(OR₅)₂ un cycle 1,3-dioxolane ou 1,3-dioxane et Y représente soit un atome d'hydrogène, soit un groupe 1,1-diméthyléthyle, soit un groupe triphénylméthyle, soit un groupe triméthylstannyle, soit un groupe tributylstannyle, soit un groupe (1,1-diméthyléthyl)diméthylsilyle, soit un groupe (1,1-diméthyléthyl)diphénylsilyle, soit un groupe 2-cyanoéthyle, soit un groupe CH₂OR₆ où R₆ est un groupe méthyle, phénylméthyle, 1,1-diméthyléthyle, 2,2,2-trichloroéthyle, benzyloxycarbonyle ou 2,2,2-trichloroéthyloxycarbonyle,
Y étant en position 1 ou 2 du cycle tétrazole.

Des dérivés de [1,1'-biphényle]-4-carboxaldéhyde, répondant à la formule (1) 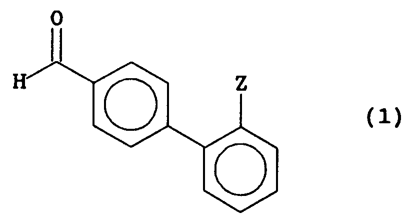
dans laquelle Z est soit un groupe cyano, soit un groupe nitro, soit un groupe alcoxycarbonyle sont mentionnés dans la demande de brevet européen EP 0449699.

Les composés de l'invention peuvent être préparés selon différentes méthodes bien connues en chimie organique. Ainsi, les aldéhydes de formule (I ter), peuvent être synthétisés, selon le schéma 1, suivant l'une ou l'autre des séquences de réactions, en fonction de la nature du substituant Y : 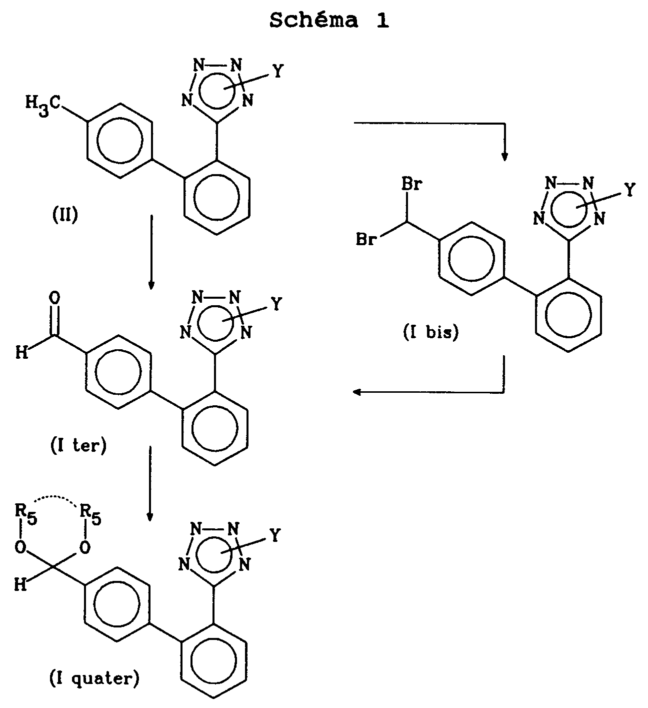
Dans une première méthode, on traite un dérivé de formule (II) avec un agent oxydant, comme par exemple le nitrate d'ammonium cérique ou le permanganate de potassium. On trouvera des exemples d'utilisation de cette méthode dans *Synthesis 1989, 293* et dans *Can. J. Chem. 1976, 54, 411*. Les dérivés de formule (II) dans laquelle Y est soit un atome d'hydrogène, soit un groupe Sn(R)₃ où R est un groupe (C₁₋₆)alkyle ou un groupe phényle ou un groupe cyclohexyle, soit un groupe triphénylméthyle, soit un groupe 2-cyanoéthyle, soit un groupe (4-nitrophényl)méthyle sont décrits dans la demande de brevet européen EP 0291969.

Dans une deuxième méthode, on transforme les dérivés de formule (II) en dérivés de formule (I bis), par exemple en les faisant réagir avec du N-bromosuccinimide, dans un solvant tel que le tétrachlorure de carbone, en présence d'un initiateur tel que le peroxyde de benzoyle ou l'α,α'-azoisobutyronitrile, à la température de reflux. On transforme les dérivés de formule (I bis) en aldéhydes de formule (I ter). Cette transformation peut être réalisée, soit en faisant réagir une amine telle que la pyridine, l'hydroxylamine, l'hydrazine ou la morpholine et en faisant une hydrolyse, soit par une hydrolyse catalysée par un acide ou une base organique ou inorganique, soit par une hydrolyse catalysée par des sels d'argent, soit par une solvolyse dans un alcool ou un diol aliphatique. On trouvera des exemples de ces types de transformations dans *J. Labelled Comp. 1972, 8, 397; Syn. Comm. 1987, 17, 1695; Org. Synth. 1954, 34, 82; Tetrahedron Lett. 1984, 25, 3099*.
La fonction aldéhyde des composés de formule (I ter) peut être protégée, par exemple, sous forme d'acétal aliphatique ou alicyclique, pour donner des composés de formule (I quater), en particulier des 1,3-dioxolanes ou 1,3-dioxanes, en utilisant des techniques bien établies en chimie organique et décrites, par exemple, par T.W. GREENE dans *Protective Groups in Organic Synthesis*, *1981, Ed. Wiley-Interscience*.

Le brevet US 5039814 décrit un procédé de préparation de dérivés analogues, à partir d'organolithiens qui inclut une étape intermédiaire de transmétallation en présence d'un catalyseur.
Un autre procédé de l'invention permet un couplage direct aryle-aryle en présence d'un catalyseur, selon le schéma 2 de la page suivante :

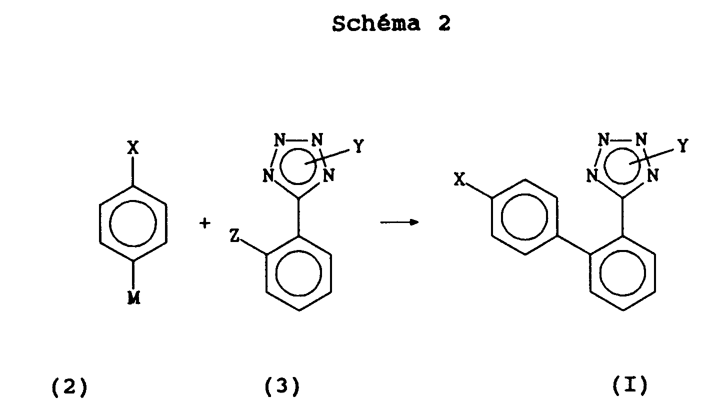

Pour les composés de formule (I) dans laquelle X ne représente pas le groupe dibromométhyle, on réalise un couplage entre un composé de formule (2) dans laquelle M représente un métal choisi parmi l'aluminium, le bore, le cadmium, le cuivre, le magnésium et le zinc et un composé de formule (3) dans laquelle Y est tel que défini précédemment et Z représente un atome de brome ou d'iode, en présence d'une quantité catalytique de palladium ou de nickel activé, si nécessaire, avec un réactif de Grignard ou un hydrure d'aluminium.

Les composés de formule (I ter) peuvent être obtenus par hydrolyse des composés de formule (I) dans laquelle X représente soit un groupe CH(OR₅)₂, soit un groupe CH(OH)OR₅, R₅ étant un atome d'hydrogène ou un groupe (C₁₋₃)alkyle qui peut éventuellement former dans le cas de CH(OR₅)₂ un cycle 1,3-dioxolane ou 1,3-dioxane.

Les composés de départ sont disponibles dans le commerce ou sont décrits dans la littérature ou peuvent être préparés selon des méthodes qui y sont décrites ou qui sont connues de l'homme du métier.
Ainsi les composés de formule (3) sont préparés selon la méthode décrite par Z. GRZONKA et coll. *J. Chem. Soc. Perkin Trans. II 1979*, **12**, 1670-1674.

Les exemples suivants illustrent l'invention. Les analyses confirment la structure des composés.

### Exemple 1

5-[4'-(dibromométhyl)[1,1'-biphényl]-2-yl]-2-(triphénylméthyl)-2*H*-tétrazole.

### 1.1 5-(4'-méthyl)[1,1'-biphényl]-2-yl]-2-(triphénylméthyl)2H-tétrazole.

### Méthode n° 1

Dans un ballon bicol, muni d'un réfrigérant et d'une ampoule d'addition, on introduit 0,360 g de magnésium dans 4 ml de tétrahydrofurane anhydre. On additionne ensuite, goutte à goutte, sous un léger reflux, 2 g (11,7 mmoles) de 1-bromo-4-méthylbenzène en solution dans 15 ml de tétrahydrofurane anhydre. On laisse agiter pendant 1 heure à la température ambiante, puis on ajoute, à 0°C, 11,7 ml d'une solution 1 M de chlorure de zinc dans l'éther éthylique. On laisse 30 minutes à la température ambiante.
On prépare le complexe de nickel dans un autre ballon en traitant 0,190 g de dichlorobis(triphénylphosphine)-nickel(II), dissous dans 10 ml de tétrahydrofurane anhydre, par 0,15 ml d'une solution 3 M de chlorure de méthylmagnésium dans le tétrahydrofurane. On additionne ensuite 3 g (5,83 mmoles) de 5-(2-iodophényl)-2-(triphénylméthyl)-2*H*-tétrazole en solution dans 15 ml de tétrahydrofurane anhydre. On agite pendant 15 minutes à la température ambiante et on introduit, par cannulation, le dérivé zincique obtenu précédemment. On agite le mélange, pendant 1 heure à la température ambiante. On additionne ensuite 20 ml d'eau, on extrait avec 150 ml d'acétate d'éthyle, on lave avec 20 ml d'une solution saturée de chlorure de sodium et on sèche sur du sulfate de magnésium.
On purifie le produit obtenu par chromatographie sur colonne de gel de silice en éluant par un mélange acétate d'éthyle/hexane (1/4).
On obtient 2,2 g de produit sous forme d'un solide blanc.
Point de fusion = 161-162 °C Rendement = 78,8 %

### Méthode n°2

Dans un ballon bicol, muni d'un réfrigérant, on introduit successivement, 1 g (1,9 mmoles) de 5-(2-iodophényl)-2-(triphénylméthyl)-*2H*-tétrazole, 0,29 g (2,13 mmoles) d'acide paratoluène boronique, 60 mg de palladiumdibenzylidène-acétone, 110 mg de triphénylphosphine, 2 ml d'une solution 2 M de carbonate de sodium et 20 ml de toluène. On porte ce mélange à 100°C pendant 16 heures. Après refroidissement et décantation, on extrait la phase aqueuse avec 150 ml d'acétate d'éthyle; on rassemble les phases organiques, on les lave successivement par 20 ml d'eau et par 20 ml d'une solution saturée de chlorure de sodium et on les sèche sur du sulfate de magnésium. Après évaporation du solvant, on purifie le résidu par chromatographie sur colonne de gel de silice en éluant par un mélange acétate d'éthyle/hexane (1/4).
On obtient 0,69 g de produit sous forme d'un solide blanc.
Point de fusion = 161-162 °C Rendement = 74,1 %

### Méthode n° 3

Dans un ballon bicol, muni d'un réfrigérant et d'une ampoule d'addition, on introduit 0,360 g de magnésium dans 4 ml de tétrahydrofurane anhydre. On additionne ensuite, goutte à goutte, sous un léger reflux, 2 g (11,7 mmoles) de 1-bromo-4-méthylbenzène en solution dans 10 ml de tétrahydrofurane anhydre. On laisse agiter pendant 1 heure à la température ambiante.
On prépare le complexe de palladium dans un autre ballon en traitant 0,2 g de dichlorobis(triphénylphosphine)palladium (II), dissous dans 10 ml de tétrahydrofurane anhydre, par 0,6 ml d'une solution 1 M d'hydrure de diisobutylaluminium dans l'hexane. On ajoute à cette solution 3 g (5,8 mmoles) de 5-(2-iodophényl)-2-(triphénylméthyl)-2*H*-tétrazole en solution dans 15 ml de tétrahydrofurane anhydre. On agite pendant 15 minutes à la température ambiante et on introduit, par cannulation, le dérivé magnésien obtenu précédemment. On agite le mélange, pendant 1 heure à la température ambiante. On additionne ensuite 15 ml d'eau, on extrait avec 100 ml d'acétate d'éthyle, on lave avec 20 ml d'une solution saturée de chlorure de sodium et on sèche sur du sulfate de magnésium.
On purifie le produit obtenu par chromatographie sur colonne de gel de silice en éluant par un mélange acétate d'éthyle/hexane (1/4).
On obtient 1,6 g de produit sous forme d'un solide blanc.
Point de fusion = 161-162 °C Rendement = 57,3 %

### 1.2 5-[4'-(dibromométhyl)[1,1'-biphényl]-2-yl]-2-(triphénylméthyl)-2H-tétrazole.

On dissout 5 g (10,5 mmoles) du composé obtenu précédemment dans 60 ml de tétrachlorure de carbone. On ajoute 4,1 g (23 mmoles) de N-bromosuccinimide et 50 mg (0,304 mmole) d'α,α'-azobisisobutyronitrile, puis on porte au reflux pendant 2 heures. On laisse refroidir et on filtre. On évapore et on triture le résidu sous éther. On obtient 5,97 g du composé attendu sous forme de poudre blanche.
Ce composé est utilisé par la suite sans purification.
Rendement = 89 % Point de fusion = 176°C

### Exemple 2

### 5-[4'-(dibromométhyl)[1,1'-biphényl]-2-yl]-2-(1,1-diméthyléthyl)-2H-tétrazole.

### 2.1 5-(4'-méthyl)[1,1'-biphényl]-2-yl]-2-(1,1-diméthyléthyl)-2H-tétrazole.

On dissout 2,3 g (8,8 mmoles) de 5-(4'-méthyl)[1,1'-biphényl]-2-yl]-1*H*-tétrazole dans 10 ml d'acide trifluoroacétique, on ajoute 0,86 g (8,8 mmoles) d'acide sulfurique à 95 % et 1,3 g (18 mmoles) de tertiobutanol dissous dans 2 ml de dichlorométhane. On agite pendant 5 heures à la température ambiante. On verse le milieu réactionnel dans 120 ml d'eau glacée et on extrait par 2 fois 80 ml de dichlorométhane. On lave la phase organique par 50 ml d'une solution saturée en bicarbonate de sodium. On sèche sur du sulfate de magnésium. On évapore le solvant pour obtenir 2,82 g du composé attendu sous forme d'huile qui se solidifie. Le produit brut est utilisé tel quel par la suite. Par cristallisation dans un mélange éthanol/eau, on obtient le produit sous forme d'une poudre blanche.
Rendement quantitatif Point de fusion = 93-95°C

### 2.2 5-[4'-(dibromométhyl)[1,1'-biphényl]-2-yl]-2-(1,1-diméthyléthyl)-2H-tétrazole.

Selon l'exemple 1.2, à partir de 2,2 g (7,5 mmoles) du composé précédemment obtenu, on obtient une huile que l'on chromatographie sur colonne de gel de silice en éluant par un mélange dichlorométhane/cyclohexane. On triture l'huile purifiée dans du pentane et on obtient 1,57 g du composé attendu sous forme de poudre blanche. Rendement = 46 % Point de fusion = 92-94°C

### Exemple 3

2'-[2-(triphénylméthyl)-2*H*-tétrazol-5-yl][1,1'-biphényle]-4-carboxaldéhyde.

On suspend 5,4 g (7,2 mmoles) du composé obtenu en 1.2 dans un mélange contenant 100 ml d'acétonitrile et 10 ml de diméthylformamide. On ajoute 4,18 g (15 mmoles) de carbonate d'argent et on chauffe au reflux pendant 7 heures. On dilue le milieu par 100 ml de dichlorométhane et on filtre le précipité. On évapore le solvant. Le produit brut est repris par 150 ml de dichlorométhane et lavé par 2 x 50 ml d'une solution de carbonate de potassium 0,5 M, puis par 50 ml d'eau, puis par 50 ml d'acide chlorhydrique 0,1 N et finalement par 50 ml d'eau. On sèche la phase organique sur du sulfate de magnésium et on évapore le solvant. On purifie l'huile obtenue par chromatographie sur colonne de gel de silice, en éluant par un gradient de dichlorométhane/cyclohexane. On obtient une gomme incolore que l'on triture dans l'éther. On obtient 1,4 g du composé attendu sous forme de poudre blanche.
Rendement = 40 % Point de fusion = 157-158°C

### Exemple 4

2'-[2-(1,1-diméthyléthyl)-2*H*-tétrazol-5-yl][1,1'-biphényle]-4-carboxaldéhyde.

A partir de 1,2 g (2,7 mmoles) du composé obtenu en 2.2, selon le protocole décrit dans l'exemple 3, on obtient 0,8 g de composé attendu sous forme d'une huile. On la purifie par chromatographie sur colonne de gel de silice en éluant avec du dichlorométhane. On triture l'huile purifiée dans du pentane et on obtient 0,59 g de produit sous forme de poudre blanche. Après recristallisation dans un mélange éthanol/eau, on obtient le produit sous forme de cristaux blancs.
Rendement = 72 % Point de fusion = 64-67°C
Ce composé peut également être obtenu en chauffant, à la température de reflux, 0,05 g (0,11 mmole) du composé obtenu en 2.2, dans 10 ml de méthanol, pendant 18 heures. Après évaporation du solvant, on purifie le produit par chromatographie sur colonne de gel de silice en éluant par un mélange acétate d'éthyle/heptane (1/4). On obtient 0,02 g du composé attendu.
Rendement = 50 %

### Exemple 5

5-[4'-(diméthoxyméthyl)[1,1'-biphényl]-2-yl]-2-(triphénylméthyl)-2*H*-tétrazole.

Dans un ballon bicol, muni d'un réfrigérant et d'une ampoule d'addition, on introduit 0,285 g de magnésium dans 4 ml de tétrahydrofurane anhydre. On additionne ensuite, goutte à goutte, sous un léger reflux, 2 g (8,6 moles) de 1-bromo-4-(diméthoxyméthyl)benzène en solution dans 10 ml de tétrahydrofurane anhydre. On laisse sous agitation pendant 1 heure à la température ambiante, puis on ajoute, à 0° C, 11,6 ml d'une solution 1 M de chlorure de zinc dans l'éther éthylique. On laisse 30 minutes à la température ambiante. On prépare le complexe de palladium dans un autre ballon en traitant 0,205 g de dichlorobis(triphénylphosphine)palladium(II), dissous dans 10 ml de tétrahydrofurane anhydre, par 0,6 ml d'une solution 1 M d'hydrure de diisobutylaluminium dans l'hexane. On ajoute à cette solution 3 g (5,8 mmoles) de 5-(2-iodophényl)-2-(triphénylméthyl)-2*H*-tétrazole en solution dans 15 ml de tétrahydrofurane anhydre. On agite pendant 15 minutes à la température ambiante et on introduit, par cannulation, le dérivé zincique obtenu précédemment. On agite le mélange, pendant 1 heure à la température ambiante. On additionne ensuite 15 ml d'eau, on extrait avec 100 ml d'acétate d'éthyle, on lave avec 20 ml d'une solution saturée de chlorure de sodium et on sèche sur du sulfate de magnésium.
On purifie le produit obtenu par chromatographie sur colonne de gel de silice en éluant par un mélange acétate d'éthyle/hexane (1/4).
On obtient 2,15 g de produit sous forme d'un solide blanc.
Point de fusion = 120-121 °C Rendement = 68,47 %

### Exemple 6

5-[4'-(diméthoxyméthyl)[1,1'-biphényl]-2-yl]-2-(1,1-diméthyléthyl)-2*H*-tétrazole.

Dans un ballon bicol, muni d'un réfrigérant et d'une ampoule d'addition, on introduit 0,61 g de magnésium dans 4 ml de tétrahydrofurane anhydre. On additionne ensuite, goutte à goutte, sous un léger reflux, 4,26 g (18,4 mmoles) de 1-bromo-4-(diméthoxyméthyl)benzène en solution dans 15 ml de tétrahydrofurane anhydre. On laisse agiter pendant 1 heure à la température ambiante, puis on ajoute, à 0 °C, 22 ml d'une solution 1 M de chlorure de zinc dans l'éther éthylique. On laisse 30 minutes à la température ambiante.
On prépare le complexe de nickel dans un autre ballon en traitant 0,33 g de dichlorobis(triphénylphosphine)-nickel(II), dissous dans 10 ml de tétrahydrofurane anhydre, par 0,35 ml d'une solution 3 M de chlorure de méthylmagnésium dans le tétrahydrofurane. On additionne ensuite 3,28 g (10 mmoles) de 5-(2-iodophényl)-2-(1,1-diméthyléthyl)-2*H*-tétrazole en solution dans 15 ml de tétrahydrofurane anhydre. On agite pendant 15 minutes à la température ambiante et on introduit, par cannulation, le dérivé zincique obtenu précédemment. On agite le mélange, pendant 1 heure à la température ambiante. On additionne ensuite 20 ml d'eau, on extrait avec 150 ml d'acétate d'éthyle, on lave avec 20 ml d'une solution saturée de chlorure de sodium et on sèche sur du sulfate de magnésium.
On purifie le produit obtenu par chromatographie sur colonne de gel de silice en éluant par un mélange acétate d'éthyle/hexane (1/4).
On obtient 2,46 g de produit sous forme d'un solide blanc.
Point de fusion = 59-61°C Rendement = 69,8 %

### Exemple 7

2'-(1*H*-tétrazol-5-yl)[1,1'-biphényle]-4-carboxaldéhyde.

### Méthode n° 1

On dissout 0,5 g (1 mmole) du composé obtenu dans l'exemple 3 dans 20 ml de méthanol. On ajoute 1 ml d'acide acétique et on porte au reflux. On évapore le solvant et on reprend le résidu par 60 ml de soude 1 N. On extrait la phase aqueuse par 3 x 50 ml d'éther. On filtre la phase aqueuse et on l'acidifie à pH = 1 avec de l'acide chlorhydrique concentré. On filtre le précipité et on le lave à l'eau. On obtient 0,065 g de produit sous forme de poudre blanche.
Rendement = 25 % Point de fusion = 184-186°C

### Méthode n° 2

On dissout 28 g (52 mmoles) du composé obtenu dans l'exemple 5 dans un mélange de 200 ml de tétrahydrofurane et de 70 ml d'eau. On ajoute 70 ml d'acide acétique et on porte à 50 °C pendant 4 heures. On évapore les solvants et on reprend le résidu par 200 ml de soude 1 N. On extrait la phase aqueuse par 3 fois 250 ml d'acétate d'éthyle et on l'acidifie à pH = 1 avec de l'acide chlorhydrique concentré. On filtre le précipité et on le lave à l'eau.
On obtient 8,9 g de produit sous forme d'un solide blanc.
Rendement = 68 % Point de fusion = 183-185°C

Les composés selon l'invention sont particulièrement utiles pour la préparation de différents dérivés hétérocycliques, substitués par un groupe 2'-(tétrazol-5-yl)-[1,1'-biphényle]4-méthylènyle, comme par exemple, les dérivés de 3-pyrazolone et de 4-pyrimidinone, respectivement décrits dans la demande de brevet français FR 91 02031 et dans la demande de brevet européen EP 0500409.
La synthèse est décrite dans le schéma 3 ci-dessous: 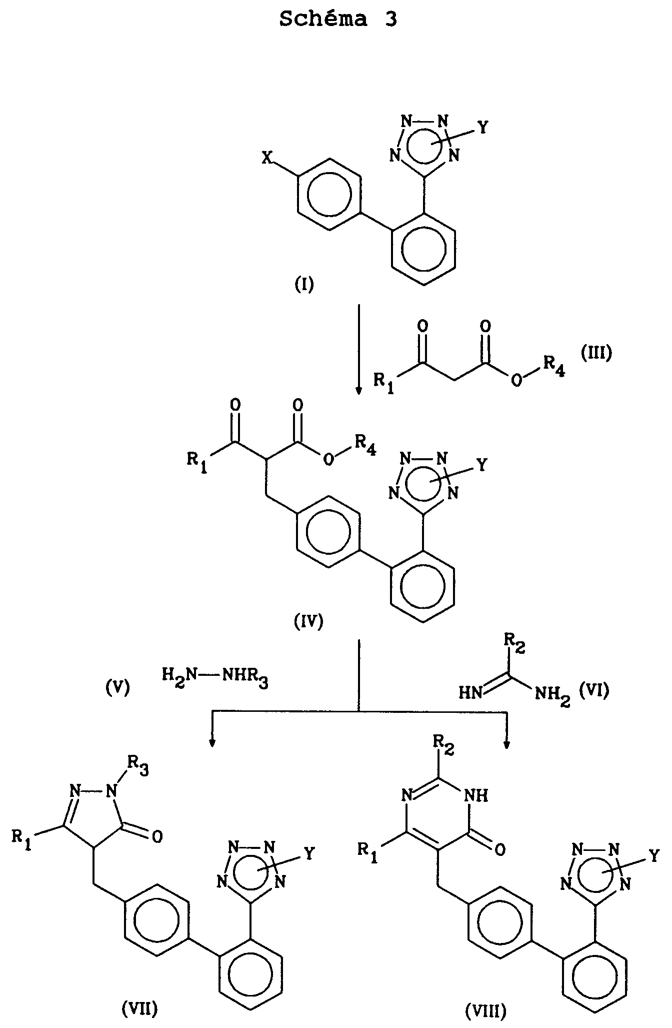
On condense un composé de formule (I) dans laquelle X et Y sont tels que définis précédemment, avec un dérivé β-cétoester de formule (III) et on réalise une hydrogénation, dans des conditions bien connues en chimie organique, conditions décrites, par exemple, dans *Org. React. 1967, 15*, 202, pour obtenir des dérivés β-cétoester de formule (IV), dérivés décrits, entre autres, dans la demande de brevet français FR 9102031 et dans la demande de brevet européen EP 0500409. On fait réagir ces composés, soit avec des hydrazines de formule (V), soit avec des amidines de formule (VI), pour préparer respectivement, des dérivés de 3-pyrazolones de formule (VII) ou des dérivés de 4-pyrimidinones de formule (VIII), comme il est décrit dans la demande de brevet français FR 9102031 et dans la demande de brevet européen EP 0500409.

L'exemple ci-dessous illustre la synthèse d'un composé de formule (IV), composé transformé en composé de formule (VII) ou (VIII) selon le schéma 3 de la page précédente.

### Synthèse du 3-oxo-2-[[2'-(2-(1,1-diméthyléthyl)-2H-tétrazol-5-yl)[1,1'-biphényl]-4-yl]méthyl]heptanoate de méthyle.

On dissout 3,6 g (12 mmoles) du composé décrit dans l'exemple 4 dans 25 ml de toluène. On ajoute 1,96 g (12 mmoles) de 3-oxo-heptanoate de méthyle, 175 mg (3 mmoles) d'acide acétique et 50 mg (0,5 mmoles) de pipéridine. On porte le mélange au reflux avec séparation azéotropique pendant 2 heures. On retire 5 ml de solvant et on remet au reflux pendant 5 heures. On ajoute 1 ml d'une solution contenant 1,2 g (20 mmoles) d'acide acétique et 0,35 g (4 mmoles) de pipéridine dans 5 ml de toluène et on porte au reflux pendant 5 heures. On évapore le solvant et on reprend le brut réactionnel par 80 ml d'éther. On lave cette solution par 40 ml d'acide chlorhydrique, puis par 2 x 40 ml de carbonate de sodium 1 M et par 40 ml d'eau. On sèche la phase organique sur du sulfate de magnésium. Après évaporation du solvant, on obtient 4,9 g de produit sous forme d'une huile jaune. On reprend l'huile obtenue par 80 ml de méthanol, on ajoute 350 mg de charbon palladié et on hydrogène à l'appareil de Parr pendant 3 heures. On filtre le catalyseur et on évapore le solvant. Le brut réactionnel est purifié par chromatographie sur colonne de gel de silice en éluant par un gradient d'acétate d'éthyle et d'heptane. On obtient 3,6 g d'une huile jaune pâle.
Rendement = 69 %
RMN ¹H (CDCl₃, δ en ppm par rapport au TMS): 8,00-7,92, m, 1H, arom; 7,64-7,43, m, 3H, arom; 7,15, s, 4H, arom; 3,79, t, 1H, 7,7 Hz,>CH-; 3,69, s, 3H, OMe; 3,15, d, 2H, 7,7 Hz, CH₂-Ar; 2,64-2,25, m, 2H, CH₂-C(O); 1,59, s, 9H, (CH₃)₃; 1,62-1,41, m, 2H, CH₂; 1,36-1,15, m, 2H, CH₂; 0,87, t, 3H, CH₃.
IR (NaCl, film): 1750,1720 cm⁻¹

Dans les formules (III) à (VIII),
R₁ représente soit un groupe (C₁ ₋ ₇)alkyle droit ou ramifié, soit un groupe (C₃ ₋ ₉)alcényle droit ou ramifié, soit un groupe cyclo(C₃ ₋ ₇)alkyl(C₁ ₋ ₆)alkyle,
R₂ représente soit un atome d'hydrogène, soit un groupe (C₁ ₋ ₇)alkyle droit ou ramifié, soit un groupe (C₃ ₋ ₉)alcényle droit ou ramifié, soit un groupe (C₃ ₋ ₉)alcynyle droit ou ramifié, soit un groupe (C₁ ₋ ₇)alcoxy droit ou ramifié, soit un groupe cyclo(C₃ ₋ ₇)alkyl(C₁ ₋ ₃)alkyle, soit un groupe cyclo(C₃ ₋ ₇)alcoxy, soit un groupe (C₁ ₋ ₇)alkylthio droit ou ramifié, soit un groupe cyclo(C₃ ₋ ₇)alkylthio, soit un groupe aryle éventuellement substitué, soit un groupe aryloxy éventuellement substitué, soit un groupe arylthio éventuellement substitué, soit un groupe aryl(C₁ ₋ ₃)alkyle éventuellement substitué sur le noyau, soit un groupe aryloxy(C₁ ₋ ₃)alkyle éventuellement substitué sur le noyau,
soit un groupe arylthio(C₁ ₋ ₃)alkyle éventuellement substitué sur le noyau, soit un groupe hétéroaryl(C₁ ₋ ₃)alkyle éventuellement substitué sur le noyau,
R₃ représente soit un atome d'hydrogène, soit un groupe (C₁ ₋ ₇)alkyle droit ou ramifié, soit un groupe (C₃ ₋ ₉)alcényle droit ou ramifié, soit un groupe (C₃ ₋ ₉)alcynyle droit ou ramifié, soit un groupe aryle éventuellement substitué, soit un groupe aryl(C₁ ₋ ₃)alkyle éventuellement substitué sur le noyau, soit un groupe aryloxy(C₁ ₋ ₃)alkyle éventuellement substitué sur le noyau, soit un groupe arylthio(C₁ ₋ ₃)alkyle éventuellement substitué sur le noyau, soit un groupe cyclo(C₃ ₋ ₇)alkyl(C₁ ₋ ₃)alkyle, soit un groupe hétéroaryl(C₁ ₋ ₃)alkyle éventuellement substitué sur le noyau,
R₄ représente soit un groupe méthyle, soit un groupe éthyle, soit un groupe 1,1-diméthyléthyle, soit un groupe phénylméthyle.

## Revendications

1. Composés répondant à la formule générale (I) 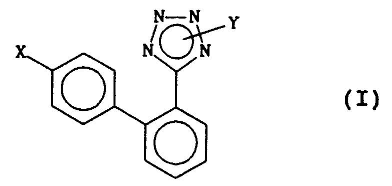 dans laquelle
X représente soit un groupe dibromométhyle, soit un groupe formyle, soit un groupe (C₁₋₄)alkyle, soit un groupe CH(OR₅)₂, soit un groupe CH(OH)OR₅, R₅ étant un atome d'hydrogène ou un groupe (C₁₋₃)alkyle qui peut éventuellement former dans le cas de CH(OR₅)₂ un cycle 1,3-dioxolane ou 1,3-dioxane et Y représente soit un atome d'hydrogène, soit un groupe 1,1-diméthyléthyle, soit un groupe triphénylméthyle, soit un groupe triméthylstannyle, soit un groupe tributylstannyle, soit un groupe (1,1-diméthyléthyl)diméthylsilyle, soit un groupe (1,1-diméthyléthyl)diphénylsilyle, soit un groupe 2-cyanoéthyle, soit un groupe CH₂OR₆ où R₆ est un groupe méthyle, phénylméthyle, 1,1-diméthyléthyle, 2,2,2-trichloroéthyle, benzyloxycarbonyle ou 2,2,2-trichloroéthyloxycarbonyle,
Y étant en position 1 ou 2 du cycle tétrazole.

2. Composés selon la revendication 1, caractérisés en ce que X représente soit un groupe dibromométhyle, soit un groupe formyle, soit un groupe diméthoxyméthyle, soit un groupe 1,3-dioxolan-2-yle, soit un groupe 1,3-dioxan-2-yle et Y représente soit un atome d'hydrogène, soit un groupe 1,1-diméthyléthyle, soit un groupe triphénylméthyle, soit un groupe méthoxyméthyle.

3. Composés selon la revendication 2, caractérisés en ce que X représente soit un groupe dibromométhyle, soit un groupe formyle et
Y représente soit un atome d'hydrogène, soit un groupe 1,1-diméthyléthyle, soit un groupe triphénylméthyle.

4. Procédé de préparation des composés de formule (I bis) 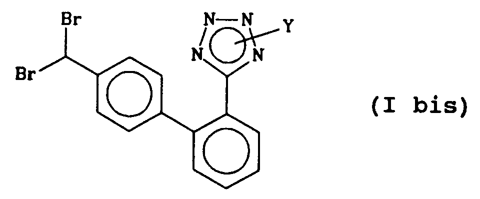 selon l'une quelconque des revendications 1 à 3,
procédé caractérisé en ce que l'on fait réagir un composé de formule (II) 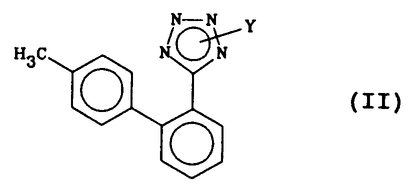 avec du N-bromosuccinimide dans des conditions de réaction radicalaire.

5. Procédé de préparation des composés de formule (I ter) 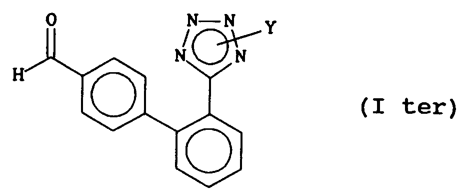 selon l'une quelconque des revendications 1 à 3,
procédé caractérisé en ce que l'on soumet un composé de formule (I bis) 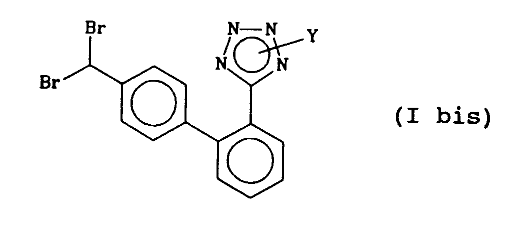 à une réaction d'hydrolyse.

6. Procédé de préparation des composés de formule (I quater) 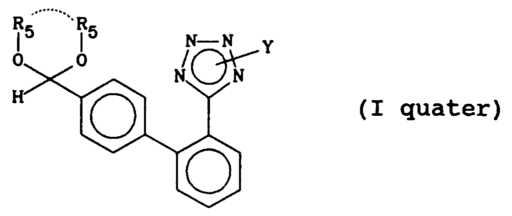 selon l'une quelconque des revendications 1 et 2,
procédé caractérisé en ce que l'on protège la fonction aldéhyde des composés de formule (I ter) 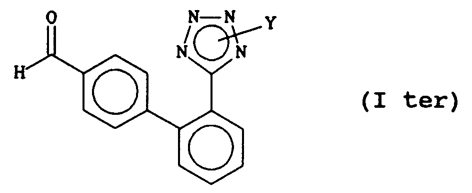 sous forme d'acétal dans des conditions classiques.

7. Procédé de préparation des composés de formule (I) selon l'une quelconque des revendications 1 à 3 pour lesquels X ne représente pas un groupe dibromométhyle,
procédé caractérisé en ce que l'on fait réagir un composé de formule (2) 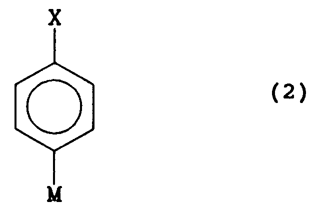 dans laquelle
X représente soit un groupe formyle, soit un groupe (C₁₋₄)alkyle, soit un groupe CH(OR₅)₂, soit un groupe CH(OH)OR₅, R₅ étant un atome d'hydrogène ou un groupe (C₁₋₃)alkyle qui peut éventuellement former, dans le cas de CH(OR₅)₂, un cycle 1,3-dioxolane ou 1,3-dioxane et
M représente un métal choisi parmi l'aluminium, le bore, le cadmium, le cuivre, le magnésium et le zinc,
avec un composé de formule (3) 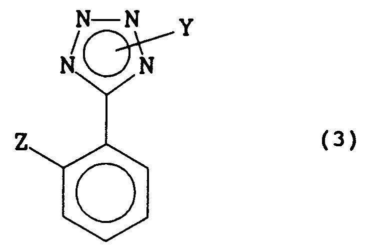 dans laquelle
Y représente soit un atome d'hydrogène, soit un groupe 1,1-diméthyléthyle, soit un groupe triphénylméthyle, soit un groupe triméthylstannyle, soit un groupe tributylstannyle, soit un groupe (1,1-diméthyléthyl)diméthylsilyle, soit un groupe (1,1-diméthyléthyl)diphénylsilyle, soit un groupe 2-cyanoéthyle, soit un groupe CH₂OR₆ où R₆ est un groupe méthyle, phénylméthyle, 1,1-diméthyléthyle, 2,2,2-trichloroéthyle, benzyloxycarbonyle ou 2,2,2-trichloroéthyloxycarbonyle,
Y étant en position 1 ou 2 du cycle tétrazole et
Z représente un atome de brome ou d'iode,
en présence d'une quantité catalytique de palladium ou de nickel activé, si nécessaire, avec un réactif de Grignard ou un hydrure d'aluminium.

8. Procédé de préparation des composas de formule (I ter) 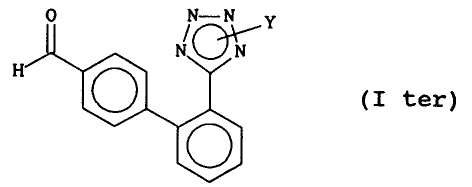 selon l'une quelconque des revendications 1 à 3,
procédé caractérisé en ce que l'on soumet un composé de formule (I) dans laquelle X représente soit un groupe CH(OR₅)₂, soit un groupe CH(OH)OR₅, R₅ étant un atome d'hydrogène ou un groupe (C₁₋₃)alkyle qui peut éventuellement former dans le cas de CH(OR₅)₂ un cycle 1,3-dioxolane ou 1,3-dioxane à une réaction d'hydrolyse.

9. Utilisation des composés selon l'une quelconque des revendications 1 à 3, pour la préparation de dérivés de 3-pyrazolone de formule (VII) et de 4-pyrimidinone de formule (VIII) dans lesquelles
R₁ représente soit un groupe (C₁ ₋ ₇)alkyle droit ou ramifié, soit un groupe (C₃ ₋ ₉)alcényle droit ou ramifié, soit un groupe cyclo(C₃ ₋ ₇)alkyl(C₁ ₋ ₆)alkyle,
R₂ représente soit un atome d'hydrogène, soit un groupe (C₁ ₋ ₇)alkyle droit ou ramifié, soit un groupe (C₃ ₋ ₉)alcényle droit ou ramifié, soit un groupe (C₃ ₋ ₉)alcynyle droit ou ramifié, soit un groupe (C₁ ₋ ₇)alcoxy droit ou ramifié, soit un groupe cyclo(C₃ ₋ ₇)alkyl(C₁ ₋ ₃)alkyle, soit un groupe cyclo(C₃ ₋ ₇)alcoxy, soit un groupe (C₁ ₋ ₇)alkylthio droit ou ramifié, soit un groupe cyclo(C₃ ₋ ₇)alkylthio, soit un groupe aryle éventuellement substitué, soit un groupe aryloxy éventuellement substitué, soit un groupe arylthio éventuellement substitué, soit un groupe aryl(C₁ ₋ ₃)alkyle éventuellement substitué sur le noyau, soit un groupe aryloxy(C₁ ₋ ₃)alkyle éventuellement substitué sur le noyau,
soit un groupe arylthio(C₁ ₋ ₃)alkyle éventuellement substitué sur le noyau, soit un groupe hétéroaryl(C₁₋₃)alkyle éventuellement substitué sur le noyau,
R₃ représente soit un atome d'hydrogène, soit un groupe (C₁ ₋ ₇)alkyle droit ou ramifié, soit un groupe (C₃ ₋ ₉)alcényle droit ou ramifié, soit un groupe (C₃₋₉)alcynyle droit ou ramifié, soit un groupe aryle éventuellement substitué, soit un groupe aryl(C₁ ₋ ₃)alkyle éventuellement substitué sur le noyau, soit un groupe aryloxy(C₁ ₋ ₃)alkyle éventuellement substitué sur le noyau, soit un groupe arylthio(C₁ ₋ ₃)alkyle éventuellement substitué sur le noyau, soit un groupe cyclo(C₃ ₋ ₇)alkyl(C₁ ₋ ₃)alkyle, soit un groupe hétéroaryl(C₁ ₋ ₃)alkyle éventuellement substitué sur le noyau,
R₄ représente soit un groupe méthyle, soit un groupe éthyle, soit un groupe 1,1-diméthyléthyle, soit un groupe phénylméthyle,
selon le schéma ci-dessous :
